# EUROPEAN PATENT SPECIFICATION

(11) **EP 0 225 807 B1**
(45) Date of publication and mention of the grant of the patent: **19.10.1994**
(21) Application number: 86309622.8
(22) Date of filing: 10.12.1986
(51) Int. Cl.: C12Q 1/68, C07H 21/00, C07H 19/06

(54) **Solution phase nucleic acid sandwich assay and polynucleotide probes useful therein**
Nukleinsäure-Sandwichassay in Lösungsphase und dafür geeignete Polynukleotidprobe
Essai de l'acide nucléique en phase solution et les sondes polynucléotidiques convenant pour cela

(30) Priority: 11.12.1985 US 807624
(43) Date of publication of application: 16.06.1987
(60) Divisional application: 90121584.8
(73) Proprietor: CHIRON CORPORATION, Emeryville, California 94608 (US)
(72) Inventor: Urdea, Michael Steven, Alamo California 94507 (US); Warner, Brian, Redwood City California 94062 (US); Horn, Thomas, Berkeley California 94708 (US)
(74) Representative: Hallybone, Huw George

(56) References cited:
- EP-A- 0 079 139
- EP-A- 0 139 489
- EP-A- 0 153 873
- EP-A- 0 174 879
- EP-A- 0 192 168
- EP-A- 0 209 996
- WO-A-84/03520
- WO-A-86/02929
- WO-A-86/07387
- GB-A- 2 169 403

## Description

This invention relates generally to a solution phase nucleic acid sandwich assay.

Meinkoth and Wahl, Anal. Biochem., (1984) 138:267-284, provide a review article of hybridization techniques. See also Leary et al., Proc. Natl. Acad. Sci. USA (1983) 80:4045-4049, for a description of the dot blot assay. Sandwich hybridization is described by Ranki et al., Curr. Top. Microbiol. Immunology (1983) pp. 308ff. See also Ranki et al., Gene (1983) 21:77-85, Virtanen et al., Lancet (1983) 381-383, and U.S. Patent No. 4,486,539. EPA 123,300 describes biotin-avidin complexes for use in detecting nucleic acid sequences. Sung, in Nucl. Acids Res. 9(22):6139-6151 (1981) and in J. Org. Chem. 47:3623-3628 (1982), discusses the synthesis of a modified nucleotide and application of the modified structure in oligonucleotide synthesis. Modified nucleotides are also discussed in Draper, Nucleic Acids Res. 12:2:989-1002 (1984), wherein it is suggested that cytidine residues in RNA be modified so as to bind to reporter molecules. Later work suggests similar modification of cytidine residues in DNA (Anal. Chem. 157(2):199 (1986). European Patent Application 063879, filed 6 April 1982, and PCT Application No. PCT/US84/00279 also describe modified nucleotides and applications thereof.

The increasing ease of cloning and synthesizing DNA sequences has greatly expanded opportunities for detecting particular nucleic acid sequences of interest. No longer must one rely on the use of immunocomplexes for the detection of pathogens, lesions, antigens, and the like. Rather than detecting particular determinant sites, one can detect DNA sequences or RNA sequences associated with a particular cell. In this manner, diseases can be diagnosed, phenotypes and genotypes can be analyzed, as well as polymorphisms, relationships between cells, and the like.

For the most part, analyses of DNA sequences have involved the binding of a sequence to a solid support and hybridization of a complementary sequence to the bound sequence. The annealing and complexing step usually involves an extended period of time and requires careful washing to minimize non-specific background signals. There is substantial interest in developing new techniques for analyzing nucleic acid sequences, which are more rapid, minimize the number of manipulative steps, and provide for an increased signal to noise ratio.

Copending European Patent Application No 90121584.8, which is divided from the present application is directed to polynucleotide probes useful in such techniques. The majority of polynucleotide probes in current use are radioactively labeled, e.g. with isotopes of hydrogen (³H), phosphorus (³²P), carbon (¹⁴C) or iodine (¹²⁵I). These materials are relatively simple to synthesize by direct inclusion of the radioactive moieties, e.g. by kinasing with ³²P-labeled ATP, equilibrating with tritiated water, or the like. As is well known, however, use of such radioactive labels has drawbacks, and other detectable species which are not radioactive are preferred.

In order to incorporate other, non-radioactive types of detectable species in a nucleotide, some sort of chemical modification of the nucleotide is required. It is widely recognized that nucleotide modification is a difficult and sensitive procedure, as any modification reaction has to be mild enough to leave the RNA or DNA molecules intact, while giving a modified nucleotide product which can participate in normal base pairing and stacking interactions. These considerations typically limit nucleotide substitution positions to the 5-position of a pyrimidine and the 8-position of a purine, as noted in the literature (see, e.g., European Patent Application 063879, cited supra).

Other considerations must also be taken into account. Base pairing may be hindered during hybridization if the detectable label is at one end of the nucleotide chain rather than present at some point within it. Further, it has proved difficult to provide even non-radioactively labeled probes which may be inexpensively synthesized in large quantity. Thus, many known probes are limited in their potential applications.

Methods and compositions are provided by the present invention for detecting particular nucleic acid sequences. Two sets of reagents are employed, which are referred to as the capturing set and the labeling set. Each set has at least two members. The labeling set has (1) a first probe set, which comprises one or a group of first analyte complementary sequence-first label reagent recognition sequence conjugate(s); and (2) one or a group of sequences complementary to said first recognition sequence-label conjugate(s). The capturing set has (1) a second probe set, which comprises one or a group of second analyte complementary sequence(s) joined to second capturing reagent polynucleotide recognition sequence(s); (2) one or a group of sequences complementary to said second capturing recognition sequence(s) bound to a separation member or preferably a first specific binding pair member to define the capturing conjugate; and (3) a separation member joined to a first complementary specific binding pair member when (2) does not have the separation member.

The single stranded nucleic acid sample may be joined with the probes containing the complementary sequences of the two sets under annealing conditions, followed by the addition of the capturing and optionally the labeling conjugates to provide for the analyte complex with the specific binding pair member and optionally the label. The probe hybridized analyte sequence is separated by combining the complex with the separating means and separating probe bound analyte from unbound analyte. Where the label has not been previously added, the first recognition sequence-label conjugate is added to the phase containing the separation member under hybridizing conditions. The label may then be detected in either phase.

The aforementioned copending European Patent Application No 90121584.8 describes modified derivatizable nucleotides having the structure of Formula 1, and their preparation including the step of derivatising the R¹ moiety with a detectable label: 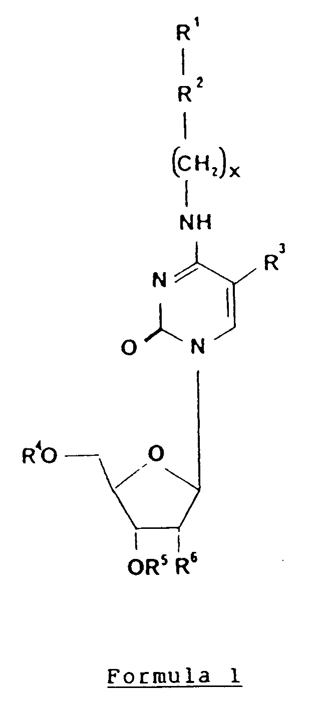
wherein R¹ is a reactive group derivatizable with a detectable label, which reactive group may be amine, carboxyl or thiol and further may be protected for various synthetic manipulations, R² is an optional linking moiety such as those typically used to label proteins, and includes an amide, thioether or disulfide linkage or a combination thereof, R³ is selected from the group consisting of hydrogen, methyl, bromine, fluorine and iodine, R⁴ is hydrogen, an anchoring group which covalently binds the structure to a solid support, or a blocking group such as dimethoxytrityl or pixyl, which blocking group is generally base-stable and acid-sensitive, R⁵ is hydrogen, an anchoring group which covalently binds the structure to a solid support, or a phosphorus derivative enabling addition of nucleotides at the 3' position, and may be, for example, PO₃H₂, a phosphotriester, a phosphodiester, a phosphite, a phosphoramidite, H-phosphonate or a phosphorothioate, and R⁶ is H, OH, or OR where R is a functional group useful as a protecting moiety in RNA synthesis, and x is an integer in the range of 1 and 8 inclusive.

The copending application also describes nucleic acid probes using one or more of the above modified nucleotides. The probe can be used to screen a sample containing a plurality of single-stranded or double-stranded polynucleotide chains and will label the desired sequence, if present, by hybridization.

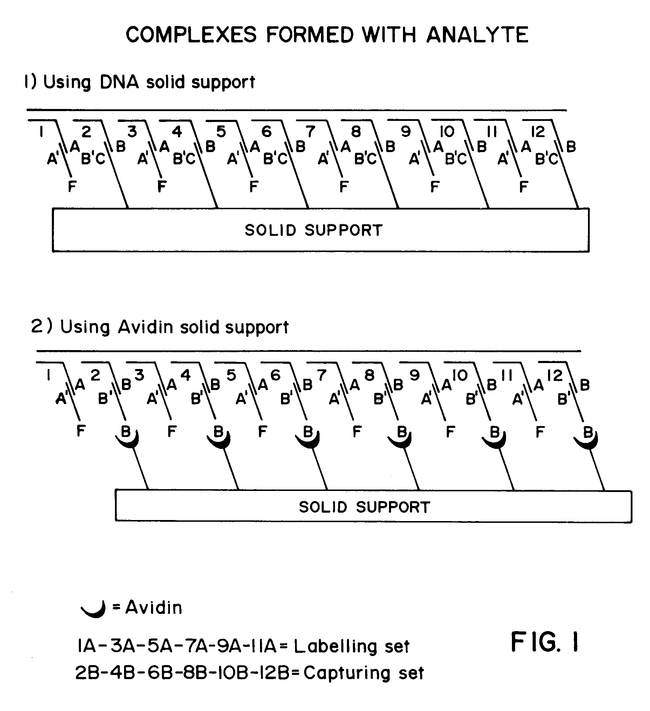

In the accompanying drawing:-
Figure 1 is an illustrative depiction of a complex from the various components bound to a solid support (1) using DNA bridges for non-covalent binding and (2) using biotin-avidin bridges for non-covalent binding.

### 1. Sandwich Assay Method

Methods and compositions are provided for detecting a nucleic acid sequence by employing two sets of reagents. By using combinations of nucleic acid sequences complementary to a nucleic acid analyte and to arbitrary sequences and specific binding pair members, a detectable label may be separated into two phases in proportion to the amount of analyte present in a sample. By providing for annealing of nucleic acid sequences in solution, the time for performing the assay can be substantially diminished as compared to annealing on a solid surface and the number of separations and washing steps required can be limited and be less critical, so as to reduce technician error. Reagents containing complementary sequences can be added in excess during or at the end of the denaturation to inhibit renaturation of double stranded DNA and to react rapidly with the analyte strand by diffusion in solution. The rate of binding to the solid support can also be accelerated by the presence of a large amount of the binding pair member bound to the support. In addition, by adding the label conjugate as the last reagent, the analyte will be present in a highly concentrated form.

As indicated above, the method involves two sets of reagents. The first set results in labeling the analyte sequence. The second set provides the means for separating label bound to analyte from unbound label in the assay medium.

The first set, the labeling set, will involve at least two reagents and may involve 10 to 30 reagents or more. The first reagent will be a subset of nucleic acid reagents and each member of the subset will have two nucleic acid regions. The first nucleic acid region of each member of the subset will be a region complementary to a sequence of the analyte. The second nucleotide sequence will be a recognition site for the labeling reagent. This second sequence will be selected, so as not to be encountered by endogenous sequences in the sample.

The subsets will have regions complementary to the analyte sequence of at least 15 nucleotides (nt), usually at least 25nt, more usually at least 50nt, and not more than about 5kb, usually not more than about 1kb, preferably not more than about 100nt. The sequence complementary to the analyte may be joined to a non-specific sequence at either or both the 5ʹ and 3ʹ-termini. The non-complementary sequence, if judiciously selected so as not to bind to sequences in the assay which could result in false positives, can be of any length, usually fewer than 10kb, more usually fewer than 5kb.

The complementary sequences will be chosen so as to leave areas for binding of the other reagents to the analyte. Usually, areas of at least 25nt will be left available, where the analyte sequences complementary to the sequences of the individual members of the reagent subset may be substantially contiguous or separated and members of one subset may alternate with members of the other subset. The particular pattern of binding between the two subsets may vary widely depending on the sequences of the analyte.

The reagent sequences may be prepared by synthesis in accordance with conventional procedures or by cloning and may be modified as appropriate for labeling.

The set of sequences which are complementary to the analyte may be selected based on a variety of considerations. Depending upon the nature of the analyte, one may be interested in a consensus sequence, a sequence associated with polymorphisms, a particular phenotype or genotype, a particular strain, or the like. Thus, the labelling complementary sequences will be chosen in conjunction with the other complementary sequences of the capturing set to provide information concerning the analyte.

The labeled sequence will include a sequence complementary to the first recognition sequence of the labeling probe(s). The labeling sequence will include one or more molecules, which directly or indirectly provide for a detectable signal. The labels may be bound to individual members of the complementary sequence or may be present as a terminal member or terminal tail having a plurality of labels. Various means for providing labels bound to the sequence have been reported in the literature. See, for example, Leary et al., Proc. Natl. Acad. Sci. USA (1983) 80:4045; Renz and Kurz, Nucl. Acids Res. (1984) 12:3435; Richardson and Gumport, Nucl. Acids Res. (1983) 11:6167; Smith et al., Nucl. Acids Res. (1985) 13:2399; Meinkoth and Wahl, Anal. Biochem. (1984) 138:267. The labels may be bound either covalently or non-covalently to the complementary sequence.

Labels which may be employed include radionuclides, fluorescers, chemiluminescers, dyes, enzymes, enzyme substrates, enzyme cofactors, enzyme inhibitors, enzyme subunits, metal ions, and the like. Illustrative specific labels include fluorescein, rhodamine. Texas red, phycoerythrin, umbelliferone, luminol, NADPH, α-β-galactosidase, horseradish peroxidase, etc.

The labeled sequence can be conveniently prepared by synthesis. By providing for a terminal group which has a convenient functionality, various labels may be joined through the functionality. Thus, one can provide for a carboxy, thiol, amine, hydrazine or other functionality to which the various labels may be joined without detrimentally affecting duplex formation with the sequence. As already indicated, one can have a molecule with a plurality of labels joined to the sequence complementary to the labeling sequence. Alternatively, one may have a ligand bound to the labeling sequence and use a labeled receptor for binding to the ligand to provide the labeled analyte complex.

The second set of reagents provides the means for separation of label bound to analyte from unbound label. The means for the separation or capturing means involves at least one capturing probe, usually a plurality of probes defining a subset, which includes two polynucleotide sequence regions that include a second subset of sequence complementary to the analyte, differing from the first subset of complementary sequences of the labeling probe and a recognition sequence, different from the first subset recognition sequence of the labeling probe. The second set of recognition sites for the capture probes may lie between the first set of recognition sites for the labeling probes as described above. The capturing sequences will be selected and synthesized in the same manner as described above using the considerations directing the selection for the labeling probes. Thus, the same constraints will be involved in preparing the capturing probes.

While the separating means may be directly bound to a sequence complementary to the capturing recognition sequence, preferably a specific binding pair member will be bound to the complementary sequence. The specific binding pair member will be a ligand or receptor, preferably a ligand. Ligands may be any molecules for which a naturally occurring receptor exists or can be prepared. Thus, naturally occurring ligands may be exemplified by biotin, thyroxine, enyzme substrates, steroids, and the like. Instead of naturally occurring ligands, any hapten may be employed for the production of antibodies. Ligands will generally be at least about 125 molecular weight and usually less than about 5,000 molecular weight, more usually less than about 2,000 molecular weight, and preferably less than about 1,000 molecular weight.

The receptors will generally be protein molecules and may include antibodies, naturally occurring proteins, such as avidin, thyroxine binding globulin, etc., lectins, enzymes, and the like. The receptors will generally be at least about 10,000 molecular weight, more usually 12,000 or more molecular weight, usually less than about one million molecular weight.

The specific binding pair member may be joined to the second recognition sequence by any convenient means. As already indicated, the sequence may be synthesized, providing for a convenient functionality at the terminal base, which may then be used as the linkage site. One or a plurality of specific binding pair members may be joined to the complementary sequence, depending upon the particular choice of the specific binding pair member, its size, and the nature of the functionalities. Alternatively, for a large specific binding pair member, a plurality of sequences may be joined to the binding pair member. The capturing conjugate will be prepared, so that there will be little interference, if any, from the specific binding pair member with the annealing of the complementary recognition sequences and from duplex formation with the ligand-receptor binding.

Alternatively, the receptor may be an additional nucleotide sequence that specifically recognizes the recognition sequence of the capturing probe.

The separation means can be any support which allows for a rapid and clean separation of label bound to analyte from unbound label. Thus, the separation means may be particles, a solid wall surface of any of a variety of containers, e.g., centrifugal tubes, columns, microtiter plate wells, filters, tubing, etc. Preferably, particles will be employed of a size in the range of about 0.4 to 200µ, more usually from about 0.8 to 4.0µ. The particles may be any convenient material, such as latex, glass, etc.

The homologous nucleic acid sequences need not have perfect complementarity to provide homoduplexes. In many situations, heteroduplexes will suffice where fewer than 15%, usually fewer than 10% of the bases are mismatches, ignoring loops of five or more members.

Samples of analyte nucleic acids may be from a variety of sources, e.g., biological fluids or solids, food stuffs, environment materials, etc., and may be prepared for the hybridization analysis by a variety of means, e.g., proteinase K/SDS, chaotropic salts, etc. Also, it may be of advantage to decrease the average size of the analyte nucleic acids by enzymatic, physical or chemical means, e.g., restriction enzymes, sonication, chemical degradation (e.g., metal ions), etc. The fragments may be as small as O.1kb, usually being at least about O.5kb and may be 1kb or higher.

In carrying out the method, the analyte sequence will be provided in single stranded form. Where the sequence is naturally present in single stranded form, denaturation will not be required. However, where the sequence is present in double stranded form, the sequence will be denatured. Denaturation can be carried out by various techniques, such as alkali, generally from about 0.05 to O.2M hydroxide, formamide, detergents, heat, or combinations thereof. Denaturation can be carried out in the presence of the labeling probe and/or the capturing probe, so that upon change of conditions to annealing conditions, the probes will bind to any complementary sequences which are present. For example, where heat and alkali are employed, by neutralization and cooling, annealing will occur.

In many situations, it will be preferable to avoid having either the label or the separation means present during denaturation. The elevated temperatures, the non-aqueous solvents, the salts, or other materials present during denaturation may result in degradation, or undesirable modification of the label and/or separation means. Therefore, in many situations, denaturation may occur in the presence of the probes, whereupon cooling rapid annealing of the probes to the single-stranded DNA may occur, followed by the addition of the other reagents at lower temperatures and, as appropriate, under milder conditions, such as neutral pH, reduced ionic strength, or the like.

Normally, the ratio of probe to anticipated moles of analyte will be at least 1:1, preferably at least about 1.5:1, and more preferably 2:1 and may be as high as 100:1 or higher. Concentrations of each of the probes will generally range from about 10⁻⁹ to 10⁻⁶M, with sample nucleic acid concentrations varying from 10⁻²¹ to 10⁻¹²M.

After annealing conditions have been achieved, or even prior to such time, the labeled first recognition sequence and the capturing second recognition sequence are added and allowed to hybridize. Alternatively, the labeled first recognition sequence can be added after capture and separation.

A preferred embodiment which greatly reduces background and provides for extraordinarily high sensitivity will employ the following sequence. With double-stranded analyte, the analyte will be denatured in the presence of the probe or complementary sequences, or the probes may be added shortly after denaturation, and under annealing conditions. After sufficient time for annealing, the complexes may then be combined with the separation means, whereby the complexes will be bound to the support. Any background DNA or non-specifically bound DNA may be washed away so as to avoid non-specific binding of label in the next step. The solid support may then be washed to remove any non-specifically bound label to provide for a substantially reduced background of non-specifically bound label.

Consider Figure 1, part 2. In effect, the analyte which is the long bar at the top is combined with the A and B probes, where A provides the complementary sequence for the label conjugate and B provides the complementary sequence for the specific binding pair member, in this case, biotin. Thus, the A and B probes and the analyte would be joined together under annealing conditions, whereby complex formation would occur between the probes and the analyte. The biotin conjugate, Bʹ could be included with the probes or be added in a separate step to the solution containing the analyte complexes. After sufficient time for Bʹ to anneal to B, the resulting biotinylated analyte complex would then be added to the solid support to which avidin is bound. After sufficient time for the specific binding pair members to form complexes, the solid support could be washed free of any non-specific DNA, followed by the addition of the labeled sequence, which in this case is indicated as being fluorescein bound to Aʹ. The labeled sequence would be added under annealing conditions and after sufficient time for duplex formation, non-specifically bound and excess labeled conjugate would be washed away and the fluorescence of the surface determined.

A somewhat shorter protocol is provided by the configuration depicted in part 1 of Figure 1. In this situation, the probes A and B would be added to the analyte under annealing conditions, whereby analyte complexes would form. After sufficient time for analyte complexes to form, the analyte complex solution would then be added to the solid support for sufficient time for the capturing probes to bind to the solid support by complex formation with the sequence indicated as BʹC. Excess DNA could be washed away, followed by the addition of the fluorescein labeled sequence Aʹ, and the mixture allowed to anneal for sufficient time for complex formation to occur between the label and the probes. Excess in non-specifically bound label could then be washed away to provide the configuration depicted in Figure 1, part 1.

Usually, the denaturing step will take from about 5 to 25 minutes, usually from about 5 to 15 minutes, while the annealing step will generally take from about 30 minutes to 2 hours, frequency being completed in about 1 hour. Annealing can be carried out at a mildly elevated temperature, generally in the range from about 20°C to 50°C, more usually from about 25°C to 40°C, particularly 37°C.

Usually, an aqueous medium is employed, particularly a buffered aqueous medium, which may include various additives. Additives which may be employed include low concentrations of detergent (0.1 to 1%, salts, e.g., sodium citrate (0.017 to 0.170M), Ficoll, polyvinylpyrrolidone, carrier nucleic acids, carrier proteins, etc. Depending upon the nature of the specific binding pair members, various solvents may be added to the aqueous medium, such as dimethylformamide, dimethylsulfoxide, and formamide. These other solvents will be present in amounts ranging from 2 to 50%.

The stringency of the annealing medium may be controlled by temperature, salt concentration, solvent system, and the like. Thus, depending upon the length and nature of the sequence of interest, the stringency will be varied.

For the separation step, for example, using a ligand-receptor pair, the medium may be changed to optimize or approximately optimize the conditions for specific binding pair complex formation. Thus, the pH will usually be modified to be in the range of about 6 to 9, preferably about 7. This can be readily achieved, by adding from about 0.5 to 2, usually about 1 volume of about a 0.1 to 0.5M buffered medium, e.g., phosphate buffered saline, to the annealing medium. This medium may be added in conjunction with the separation means and the mixture allowed to incubate for at least 5min., usually about 10min., and less than about 60min., usually about 15 to 45min., more usually about 30min. being satisfactory.

The phases may then be separated in accordance with the nature of the separation means. For particles, centrifugation or filtration will provide for separation of the particles, discarding the supernatant or isolating the supernatant. Where the particles are assayed, the particles will be washed thoroughly, usually from one to five times, with an appropriate buffered medium, e.g., PBS. When the separation means is a wall or support, the supernatant may be isolated or discarded and the wall washed in the same manner as indicated for the particles.

Depending upon the nature of the label, various techniques can be employed for detecting the presence of the label. For fluorescers, a large number of different fluorometers are available. With enzymes, either a fluorescent or a colored product can be provided and determined fluorometrically, spectrophoto metrically or visually. The various labels which have been employed in immunoassays and the techniques applicable to immunoassays can be employed with the subject assays.

### 2. Nucleic Acid Probes

Nucleic acid probes useful in conjunction with the above assay method are probes which are prepared from one or more modified nucleotides. As noted above, these nucleotides and probes are fully described in copending European Patent Application No 90121584.8.

It is to be understood that while the invention has been described in conjunction with the preferred specific embodiments thereof, the foregoing description as well as the Examples which follow are intended to illustrate and not limit the scope of the invention. The following experimental and exemplifying material includes, inter alia and for convenience and completeness, description of nucleotide and probe preparation although these aspects are not aspects of the present invention as such. Again, the reader is referred to copending European Patent Application No 90121584.8. 
Experimental

Analyte BglII HBV Fragment

GATCTCC | TAGACACCGCCTCAGCTCTGTATCGAGAAG | CCT | TAGAGTCTCCTGAG
AGG | ATCTGTGGCGGAGTCGAGACATAGCTCTTC | GGA | ATCTCAGAGGACTC
→ 1 ← → 2

CATTGCTCACCTCACC | ATA | CTGCACTCAGGCAAGCCATTCTCTGCTGGG | GGG | AATTGATG
GTAACGAGTGGAGTGG | TAT | GACGTGAGTCCGTTCGGTAAGAGACGACCC | CCC | TTAACTAC
← → 3 ← →

ACTCTAGCTACCTGGGTGGGTA | ATA | ATTTGGAAGATCCAGCATCTAGGGATCTTG | TAG | TA
TGAGATCGATGGACCCACCCAT | TAT | TAAACCTTCTAGGTCGTAGATCCCTAGAAC | ATC | AT
4 ← → 5 ← →

AATTATGTTAATACTAACGTGGGTTTAA | AGA | TCAGGCAACTATTGTGGTTTCATATATCT
TTAATACAATTATGATTGCACCCAAATT | TCT | AGTCCGTTGATAACACCAAAGTATATAGA
6 ← → 7

T | GCC | TTACTTTTGGAAGAGAGACTGTACTTGAAT | ATT | TGGTCTCTTTCGGAGTGTGGATT
A | CGG | AATGAAAACCTTCTCTCTGACATGAACTTA | TAA | ACCAGAGAAAGCCTCACACCTAA
← → 8 ← → 9

CGCACTC | CTC | CAGCCTATAGACCACCAAATGCCCCTATCT | TAT | CAACACTTCCGGAAACT
GCGTGAG | GAG | GTCGGATATCTGGTGGTTTACGGGGATAGA | ATA | GTTGTGAAGGCCTTTGA
← → 10 ← → 11

ACTGTTGTTAGAC | GAC | GGGACCGAGGCAGGTCCCCTAGAAGAAGAA | CTCCCTCGCCTCGC
TGACAACAATCTG | CTG | CCCTGGCTCCGTCCAGGGGATCTTCTTCTT | GAGGGAGCGGAGCG
← → 12 ←

AGACGCA
TCTGCGTCTAG

→ ← indicates probed segments
 
Labelling and Capturing Probe Sets (Refer to Fig. 1)

3' GACTTGCAAGTTGGTCAAGT | ATCTGTGGCGGAGTCGAGACATAGCTCTTC | 5'
A → 1 ←

GTGGTGAAAGAGGTTTCTTC | ATCTCAGAGGACTCGTAACGAGTGGAGTGG |
B → 2 ←

GACTTGCAAGTTGGTCAAGT | GACGTGAGTCCGTTCGGTAAGAGACGACCC |
A → 3 ←

GTGGTGAAAGAGGTTTCTTC | TTAACTACTGAGATCGATGGACCCACCCAT |
B → 4 ←

GACTTGCAAGTTGGTCAAGT | TAAACCTTCTAGGTCGTAGATCCCTAGAAC |
A → 5 ←

GTGGTGAAAGAGGTTTCTTC | ATTTAATACAATTATGATTGCACCCAAATT |
B → 6 ←

GACTTGCAAGTTGGTCAAGT | AGTCCGTTGATAACACCAAAGTATATAGAA |
A → 7 ←

GTGGTGAAAGAGGTTTCTTC | AATGAAAACCTTCTCTCTGACATGAACTTA |
B → 8 ←

GACTTGCAAGTTGGTCAAGT | ACCAGAGAAAGCCTCACACCTAAGCGTGAG |
A → 9 ←

GTGGTGAAAGAGGTTTCTTC | GTCGGATATCTGGTGGTTTACGGGGATAGA |
B → 10 ←

GACTTGCAAGTTGGTCAAGT | GTTGTGAAGGCCTTTGATGACAACAATCTG |
A → 11 ←

GTGGTGAAAGAGGTTTCTTC | CCCTGGCTCCGTCCAGGGGATCTTCTTCTT |
B → 12 ←

| → Labeled Probe ← | → Probe Segments to HBV ← |
recognition sequences

A - Fluorescein label conjugate binding site
B - Biotin or DNA conjugate binding site
Label conjugate (Aʹ) for DNA or avidin support:
Fluorescein - 5ʹ CTGAACGTTCAACCAGTTCA 3ʹ
DNA sequence (BʹC) bound to solid support:
3ʹ GAAGAAACCTCTTTCACCACTGTCATCAAAAGGTTAACCATGTTTCTTGT 5ʹ
Biotin conjugate (B') for avidin support:
Biotin - 5' CACCACTTTCTCCAAAGAAG 3'

### Preparation of biotin or fluorescein labeled DNA (A' or B'):

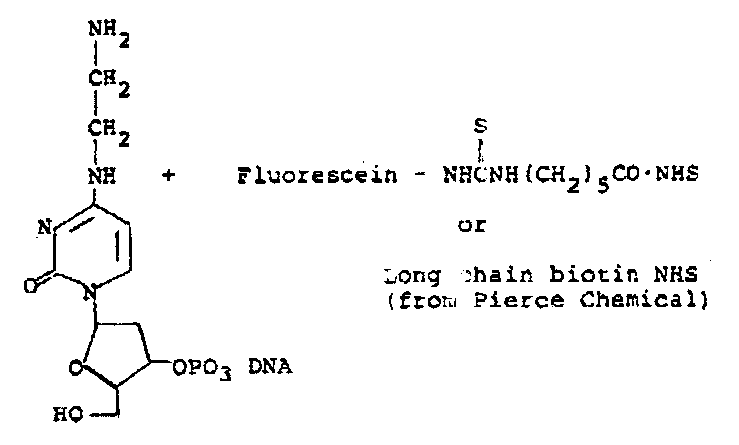

N⁴ - (2' - aminoethyl) - deoxycytosine - DNA
The analyte is an HBV BglII fragment as indicated above. (Valenzuela et al. (1981) in Animal Virus Genetics, eds. Fields, B., Jaenisch, R., Fox, C.F., Academic Press, Inc., N.Y., pp 57-70.) A subset of labeling and capturing probes are indicated, where 12 different sequences complementary to different sequences present in HBV are provided. Six of the HBV complementary sequences are joined to a common sequence (A) for complexing with the label conjugate (A'). The other six HBV complementary sequences are joined to a common sequence (B) for complexing with a biotinylated sequence (B') or a third DNA sequence (B'C) for binding to a support. In Figure 1 is shown an illustration of the final complex involving the HBV strand and the various reagents.

### Example 1

### Labeling of Caproic Acid Derivative

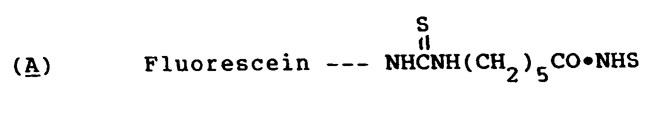

To 1 mmole of fluorescein isothiocyanate in 5 ml of DMF was added 2 mmole of 6-aminocaproic acid and 540 µl of triethylamine. After 24 h at room temperature, the product was isolated by preparative thin layer chromatography (Warner and Legg, Inorg. Chem. 18:1839 (1979)). The dried product was suspended in 10 ml of 1:1 DMF/THF (v/v) to which 1.5 mmole of N-hydroxy succinimide and 1 mmole of dicyclohexylcarbodiimide were added. After 18 h at room temperature the solution was filtered through glass wool and diluted to a 0.2M final concentration of A with DMF (assuming a 100% yield from step 1).

### Example 2

### 6-N⁴-(2-Aminoethyl)- Deoxycytidine

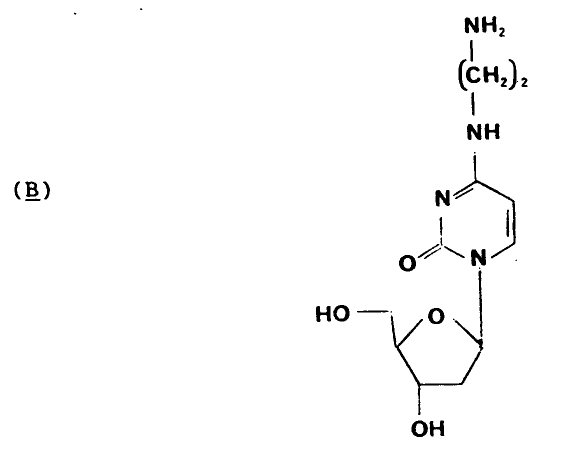

An alkylated derivative of deoxycytidine, N⁴-(2-aminoethyl) deoxycytidine (B) was prepared from properly protected deoxyuridine via the 4-tetrazoyl derivative as described by Reese and Ubasawa, Tetrahedron Lett. 21:2265 (1984). This latter derivative was converted to B by displacement of the tetrazoyl moiety with ethylene diamine essentially as described by Sung, J. Org. Chem. 47:3623 (1982) and Maggio et al., Tetrahedron Lett. 25:3195 (1984). The corresponding 5ʹ-DMT-3ʹ-phosphoramidite N⁴-(2-N-trifluoroacetylaminoethyl) deoxycytidine was prepared by blocking the alkylamine with trifluoroacetic anhydride and then preparing the corresponding N,N-diisopropyl phosphoramidite as described (Beaucage and Caruthers, supra; McBride and Caruthers, Tetrahedron Lett. 24:245 (1983)).

### Example 3

### Probe Preparation (Fluorescein Label)

Synthetic oligonucleotides were prepared by an automated phosphoramidite method as described in Warner et al., DNA 3:401 (1984). Purification was carried out according to Sanchez-Pescador and Urdea, DNA 3:339 (1984).

The aminoethyl derivative of deoxycytidine as prepared in Example 2 was incorporated by standard coupling procedures during the oligonucleotide synthesis and the purified modified oligonucleotides were used for incorporation of a fluorescein label as follows. To a dried sample (3-5 OD 260 units) of the aminoethyl deoxycytidine containing oligomer were added 50 µl of DMF and 25 µl of the 0.M2 stock solution of A described above. After 18 h at room temperature, the solution was partially purified by Sephadex G-10 chromatography eluted with water, dried and further purified by polyacrylamide gel, as above.

### Example 4

### Probe Preparation (Biotin Label)

Using the probes containing aminoethylcytidine as prepared in the previous example, biotin labeling was achieved as follows. The oligonucleotide (3-5 OD 260 units) was taken up in 50 µl 0.M1 sodium phosphate, pH 7.0 and 50 µl of DMF to which 100 µl of a DMF solution containing 1 mg of a "long chain" N-hydroxysuccinimidyl biotin (Pierce Chemical) was added. After 18 h at room temperature, the biotinylated probe was purified as described for the fluorescein labeled probe.

### Example 5

### Preparation of Solid-Supported DNA Probe

Fragment BʹC (a synthetic 50mer) was 5ʹ-phosphorylated with T4-polynucleotide kinase and ATP using standard conditions. After gel purification as described above, the oligonucleotide was dried by evacuation.

Hydroxylated latex (1Omg; 0.8µ; Pandex Laboratories) was washed with DMSO, then three portions of 40mM MES (morpholinoethanesulfonic acid), pH 6.0 by centrifugation. 1500pmoles of 5ʹ-phosphorylated fragment BʹC was taken up in 90ml of 40mM MES and added to the washed support. A solution was prepared to contain 100mg of EDAC in 100ml of MES. After adding 5µl of the EDAC solution and mixing, the reaction mixture was evaporated until 30µl total remained. The mixture was left at 37°C for 18h, then centrifuged for 2min at 12,000rpm. The supernatant was discarded. The latex was suspended in 30ml of DMSO, vortexed, 100µl of water was added, the mixture vortexed for 2min and the supernatant was discarded after centrifugation. This washing process was repeated twice. The support was then washed three times with 100ml portions of 4xSSC, H₂O, then H₂O at 37°C for 15min (yield 20 picomoles fragment BʹC per mg of latex).

### Example 6

### Assay for HBV DNA Using DNA Solid Support

A pBR322 clone containing the entire HBV genome (Valenzuela et al., Animal Virus Genetics, R. Jaenisch, B. Fields and C.F. Fox, Eds. (Academic Press: New York) pp. 57-70 (1980)) was cut with BglII and used as the analyte nucleic acid. Analyte in 10ml of formamide containing 6 picomoles of the labeling and capturing probe sets was heated to 95°C for 10min and cooled to room temperature. To this mixture, 60µl of water, 20µl of 20xSSC, 10ml of 1% NP40 and 2µl (10µg) of polyA are added, vortexed and incubated at 37°C for 1h.

The solid supported DNA probes (8 picomoles 400vg) is added and incubated for an additional 1.5h. The mixture is centrifuged at 12,000rpm for 2min and the supernatant discarded. The support is washed once by vortexing the pellet into solution with 100ml of 4xSSC, followed by centrifugation. To the washed beads are added a mixture of 4ml of 20xSSC, 2µl of 1% NP40, 1µl (5µg) polyA, 13µl of water and 6 picomoles of fluorescein labeled probe. After incubation at 37°C for 30min, the beads are transferred to a Pandex filter plate, washed four times with 100ml of 4xSSC by vacuum filtration on the O.2µ cellulose acetate membrane of the plate. The sample is vacuumed to dryness and read on the fluorescein channel A(λ_{excitation}-485; λₑₘᵢₛₛᵢₒₙ-525) of the Pandex screen machine.

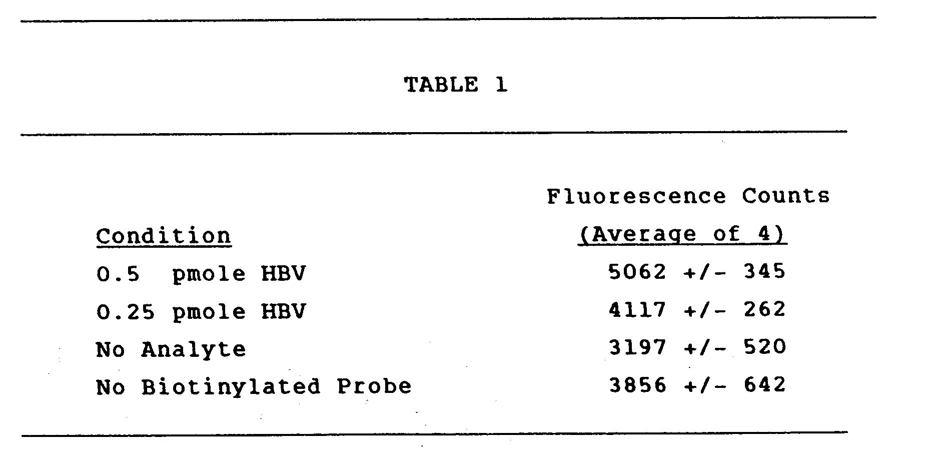

### Example 7

### Assay for HBV DNA Using Avidin Support

### Experiment 7a:

Analyte was mixed and incubated with the labeling and capturing probes as above. Biotin labeled probe (12 picomoles) in 5µl H₂O was then added, vortexed and incubated at 37°C for 30min. To the mixture, 20ml of a 0.25% (w/v) 0.8µ avidin latex (Pandex Laboratories) in 1xPBS is added and incubated at 37°C for 1h. The mixture is washed, incubated with fluorescein probe, washed and read on the Pandex screen machine as described above.

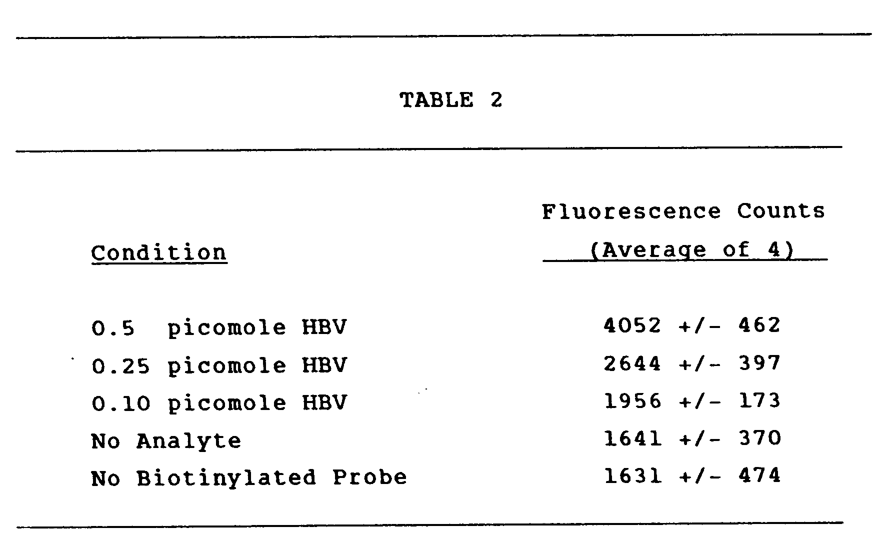

### Experiment 7b:

The HBV plasmid was sonicated to an average size of 500bp. The denaturation and hybridization were carried out as above except that 30 picomoles of labeling and capturing probes were used and a 5h annealing was employed. After incubation with 30 picomoles of biotinylated probe (2h), 50µl of 0.25% avidin beads were added and incubated (1.5h). A fluorescein probe was added and incubation was carried out for 1h followed by washing and reading on the Screen Machine as described above.

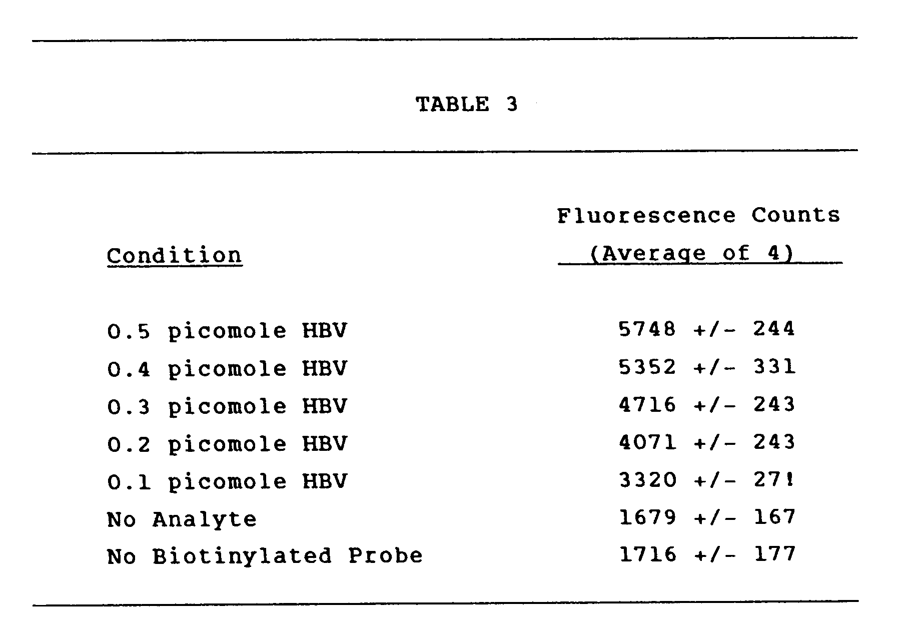

It is evident from the above results that a highly specific sensitive assay for specific nucleic acid sequence is provided. Reagents can be readily prepared to the sequence of interest and with a few simple manipulative steps, the presence or absence of a sequence in a sample determined. The method is versatile in permitting a wide variety of labels which can be readily determined by conventional equipment. Probes can be synthesized to the desired length and easily linked to the label or a support. Universal sequences can be prepared for the label and binding to the support. Various protocols may be employed where more or less rigorous removal of background interference is achieved depending upon the requirements of the assay.

### Example 8

### 5ʹ-Dimethoxytrityl-2ʹ-Deoxyuridine

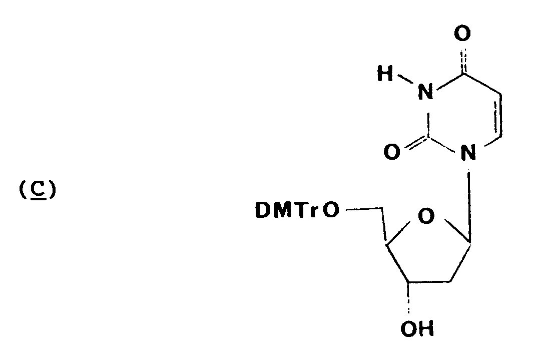

To 2-Deoxyuridine (10 g, 44 mmole) dried by coevaporation of pyridine and suspended in pyridine (100 ml) was added 18.4 g (54 mmole) 4,4ʹ-dimethoxytrityl chloride (DMT-Cl). The reaction was allowed to proceed for 18 h at room temperature, and 100 ml methanol was added to deactivate excess DMT-Cl. Most of the pyridine was then removed in vacuo, and the residue, dissolved in 500 ml ethyl acetate, was washed with saturated aqueous NaHCO₃ (3x500 ml). The organic phase was dried over solid Na₂SO₄ and evaporated to dryness. The residue was purified by flash chromatography on silica gel to give 18.0 g (77%) of 5ʹ-dimethoxytrityl-2ʹ-deoxyuridine (C).

### Example 9

### 5ʹ-O-(4,4ʹ-Dimethoxytrityl)-3ʹ-t-Butyldimethylsilyl-2ʹ-Deoxyuridine

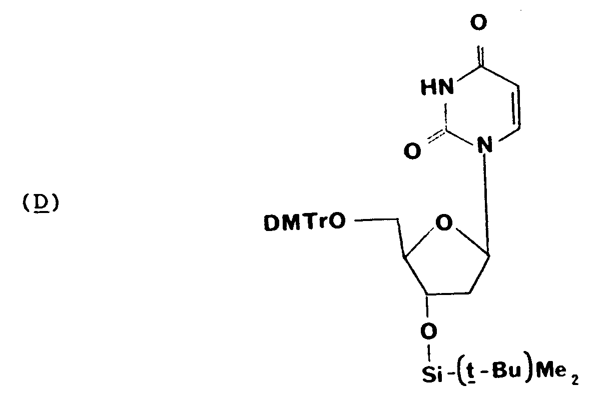

To 18 g (34 mmole) of C in 200 ml DMF was added imidazole (5.8 g, 85 mmole) with rapid stirring to assure complete dissolution. t-Butyldimethylsilyl chloride (7.65 g, 51 mmole) dissolved in a small volume of DMF was added dropwise with stirring and the reaction was allowed to proceed in the dark for 18 h at room temperature. The reaction mixture was diluted with ethyl acetate (250 ml) and extracted with NaHCO₃ (3x250 ml). The organic phase was dried over Na₂SO₄ and evaporated to dryness. The residue was purified by flash chromatography on silica gel to give 15.0 g (68% yield) of 5ʹ-O-(4,4ʹ-dimethoxytrityl-3ʹ-t-butyldimethylsilyl-2ʹ-deoxyuridine (D).

### Example 10

### 4-(1,2,3,4-Tetrazol-1-yl)-[5ʹ-(4,4ʹ-Dimethoxytrityl)-3ʹ-t-Butyldimethylsilyl-β-D-2ʹ-Deoxyribosyl] Pyridine-2(1H)-one

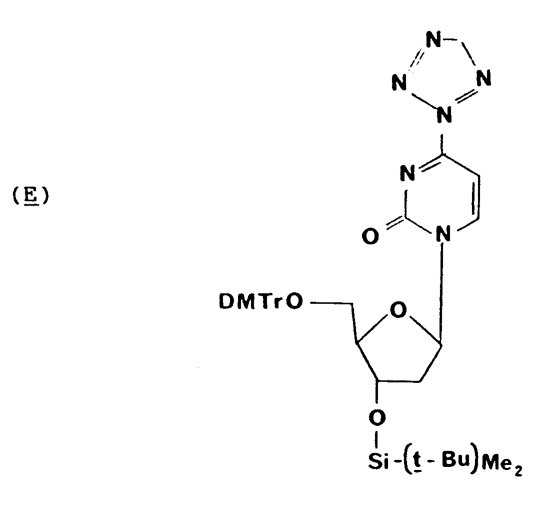

To 15.0 g (23 mmole) of D, dried by coevaporation of pyridine and dissolved in pyridine (50 ml) was added diphenylphosphate (2.9 g, 11.5 mmole) dissolved in pyridine (5 ml). 1-(Mesitylene-2-sulfonyl)-tetrazole (MS-tet) (15.5g, 61.5 mmole) dissolved in pyridine (45 ml) was added and the reaction mixture allowed to proceed in the dark for 18 h at room temperature. To the dark brown reaction mixture was added 25 ml water. After 30 min, the product was concentrated under reduced pressure. The residue was dissolved in 250 ml methylene chloride, washed with an aqueous NaHCO₃ solution (3x250 ml), dried over Na₂SO₄, and the solvent was removed under reduced pressure in the presence of toluene. The residue was purified by flash chromatography on silica gel to give 10.0 g (62%) of 4-(1,2,3,4-Tetrazol-1-yl)-[5ʹ-(4,4ʹ-dimethoxytrityl)-3ʹ-t-butyldimethylsilyl-β-D-2ʹ-deoxy-ribosyl]-pyridine-2(1H)-one (E).

### Example 11

### 4-N-(2-Aminoethyl)-5ʹ-Dimethoxytrityl-3ʹ-t-Butyldimethylsilyl-2ʹ-Deoxycytidine

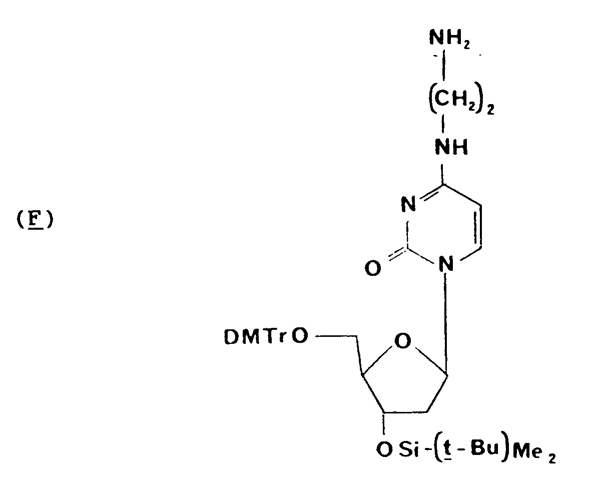

To a solution of ethylene diamine (9.3 ml, 143 mmole) in dioxane (100 ml) cooled to 5°C was added E (10.0 g, 14.3 mmole) and left for one hour. The solvent was removed at reduced pressure and the residue was coevaporated with toluene to remove excess ethylene diamine. The product was purified by chromatography on a silica gel column, eluted with 12-20% methanol in methylene chloride to give 7.15 g (75%) of 4-N-(2-aminoethyl-5ʹ-dimethoxytrityl-3ʹ-t-butyldimethylsilyl-2ʹ-deoxycytidine (F). The product was shown to react positively with ninhydrin, confirming the presence of a free amine moiety.

### Example 12

### N⁴-(N-FMOC-6-Aminocaproyl-2-Aminoethyl)-5ʹ-Dimethyltrityl-3ʹ-t-Butyldimethylsilyl-2ʹ-Deoxycytidine

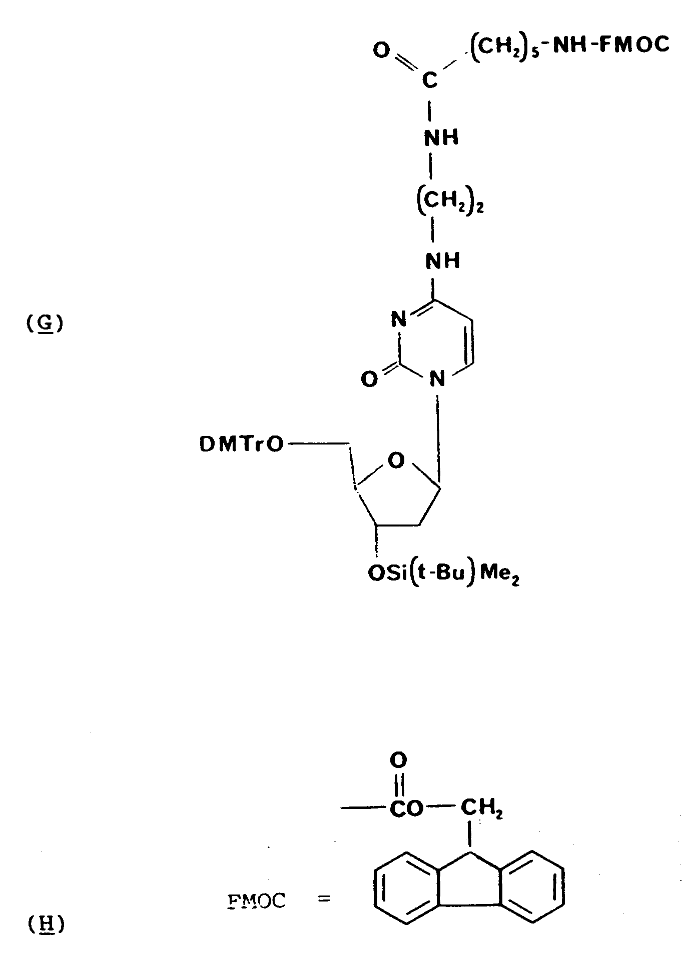

To a solution of F (6.5 g, 9.6 mmole) in pyridine (50 ml) was added N-FMOC-6-aminocaproic acid (4.26 g, 12 mmole) (FMOC represented by structure H) and DDC (2.96 g, 14.4 mmole). After 3 h, the reaction was complete as judged by tlc (silica in 10% methanol/methylene chloride). Pyridine was removed at reduced pressure. The residue was extracted with ethyl acetate, insoluble dicyclohexylurea (DCHU) filtered off and the solvent removed. The product was isolated by silica gel chromatography eluted with 4% methanol in methylene chloride affording 7.3 g (70%) of N⁴-(N-FMOC-6-aminocaproyl-2-amino-ethyl)-5ʹ-dimethyltrityl-3ʹ-t-butyldimethylsilyl-2ʹ-deoxycytidine (G).

### Example 13

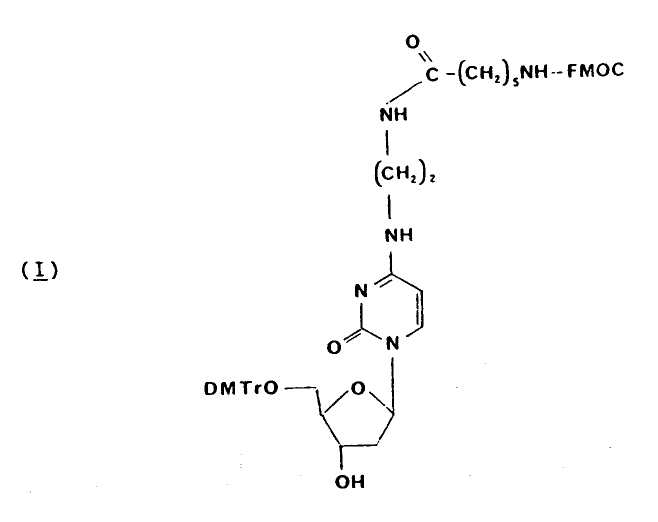

A solution of tetrabutylammonium fluoride (15 mmole, 15 ml of a 1M solution in THF) and aqueous HF (1.05 ml of a 50% aqueous solution) were mixed and dried by coevaporation of pyridine. The residue was dissolved in pyridine (15 ml) and added to G (7.2 g, 7.3 mmole) which was dissolved by sonication. After 18 hours at 4°C the reaction mixture was diluted with 200 ml methylene chloride. Concentrated aqueous NaHCO₃ was carefully added followed by solid NaHCO₃, added gradually so as to neutralize the HF/pyridine. After drying over Na₂SO₄, the organic phase was concentrated to an oil, which was subjected to silica gel chromatography. The product N⁴-(N-FMOC-6-amino caproyl-2-aminoethyl)-5ʹ-dimethoxytrityl-ʹ-deoxycytidine (I) was eluted with 5-6% methanol in methylene chloride to give an 86% yield (6.0 g).

### Example 14

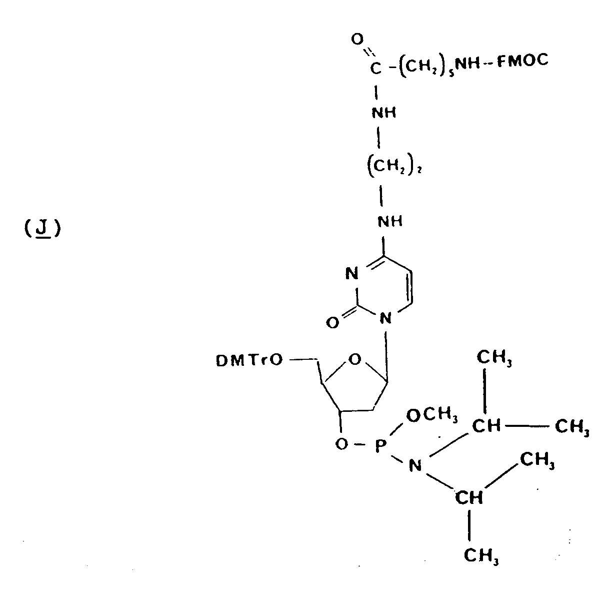

To 5.1 g (5.7 mmole) of I in methylene chloride containing (diisopropylethylamine) was added 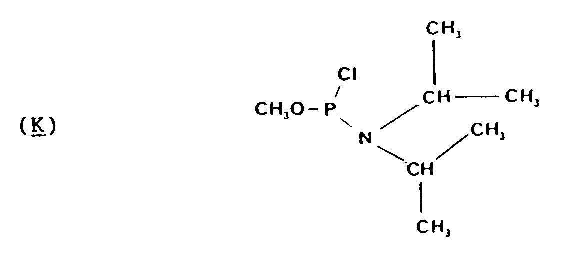
(chloro-N,N-diisopropylaminomethoxy phosphine, 1,3 ml [1.2 eq.], K) at 0°C under argon. After 1 hr, ethyl acetate (200 ml) was added and washed with 80% saturated aqueous sodium chloride; after drying of the organic phase over Na₂SO₄, the product in methylene chloride was added dropwise to hexane at -40°C to precipitate 4.43 g (75%) of J.

### Example 15

### Synthesis of Horseradish Peroxidase (HRP): DNA Conjugates

Sequence 1 (5ʹ-[LCA]CTGAACGTTCAACCAGTTCA-3ʹ) where LCA = N⁴ (6-aminocaproyl-2-aminoethyl)-deoxy cytidine) was synthesized chemically and purified as described elsewhere (Warner, et al. (1984) DNA 3, 401). To 10 OD 260 units dissolved in 50 µl of water were added 10 µl of 1.0 M sodium borate, pH 9.3, and 500 µl of distilled dimethylformamide containing 20 mg of p-phenylene diisothiocyanate. The solution was vortexed and set for 2 hr at room temperature in the dark. Approximately 3 ml of n-butanol was then added. After vortexing, adding 3 ml of water, and vortexing again, the tube was centrifuged and the yellowish upper layer discarded. The extraction process was repeated with subsequent n-butanol additions until an final volume of approximately 50 µl was obtained. The butanol was removed by evacuation, then 10 mg of HRP in 200 µl of 0.1 M borate, pH 9.3, was added. The mixture was vortexed, then set at room temperature overnight in the dark.

Separation of the HRP-DNA conjugate from free enzyme and DNA was achieved on a 7% polyacrylamide gel. The 250 µl reaction mixture was quenched with 100 µl of 25% glycerol, 0.5% SDS, 0.5% bromophenol blue, 2.5 mM EDTA. The solution was then distributed into 10 lanes of a 20 x 20 0.15 cm gel and run at 60 mAmps under standard conditions (Maxam, A., and Gilbert, W., (1980) Methods in Enzymol 65, 499-560) until the bromophenol blue was about 2/3 down the gel. The gels were set on Baker F-254 silica 60 plates that had been covered with Saran Wrap (Dow) and examined with a handheld UV-short wavelength lamp held above. Pictures of the UV-shadowed bands were taken with a Polaroid MP-4 camera system fitted with a Kodak No. 59 green filter, after which the bands were cut out with a razor blade. The bands were put into a 10-ml Bio-Rad polypropylene econo-columns to which 3 ml of 0.1 M sodium phosphate, pH 7.5, was added, then set at room temperature overnight.

The contents of the column were filtered through the frit at the column bottom into an Amicon Centricon microconcentrator that had been washed twice with distilled water. The HRP-DNA conjugate was then concentrated by centrifugation at 3500 rpm and washed twice with 1x PBS also by centrifugation. The final solution was then stored at 4°C.

### Example 16

### Assay for HBV DNA Using HRP-DNA Probe and a Biotinylated Probe Bound to an Avidin Bead

Biotin labeled probe (Bʹ; 1000 pmoles in 66.7 µl of water) was combined with 5 ml of a 0.25 % (w/v) solution of 0.8 µ avidin beads (Pandex laboratories), 1 ml of 20x SSC, 0.5 ml of 1% NP40 and 0.6 ml of 1 mg/ml polyA. After 1 h at 37°C, the beads were washed twice by centrifugation with 4x SSC, 0.1% NP40 then stored in 2.5 ml of this solution. The HBV analyte (described above) in 3 µl water was diluted into 10 µl of 4x SSC, 1% SDS, 0.5 M NaOH and 1.5 pmoles of the labeling and capturing probe sets. The mixture was heated to 95°C for 10 min., cooled on ice and neutralized with 5 µl of 1 M acetic acid, then 10 µl of the biotin probe beads were added and the solution was incubated at 37°C for 1 h.

The beads were washed twice by centrifugation with 4x SSC, 0.1% NP40, then taken up in 50 µl of 0.1% NP40, 1 mg/ml polyA, 10 mg/ml BSA, 1X PBS containing 1 pmole of HRP-DNA conjugate and set a 37°C for 1 h. The beads were washed with 0.1% NP40, 1X PBS three times then transferred in 50 µl to a microtiter dish. To each well, 50 µl of fresh OPD solution (98 mg OPD (O-phenylenediamine), 20 µl of 30% H₂O₂ in 10 ml of 50 mM sodium citrate pH 5.0) was added, mixed and set 5 min. at 37°C. The absorbances were recorded on a microtiter plate reader. Control hybridizations contained no HBV analyte.

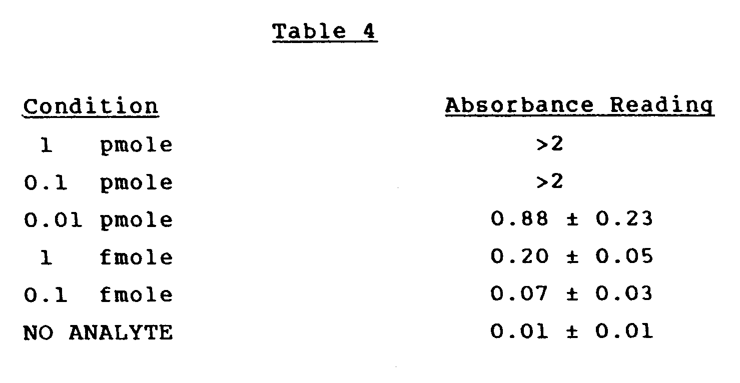

## Claims

1. An assay method for detecting a nucleic acid sequence in a sample, employing two sets of reagents: a first labeling set; and a second capturing set, said method comprising:
combining in a liquid medium under binding conditions for complementary pairs, said sample containing analyte in single-stranded form, members of said labeling set of reagents comprising:
(a) a plurality of labeling nucleic acid probes, different probes having a different first analyte complementary sequence and a first noncomplementary region comprising a label reagent recognition sequence, said first analyte complementary sequence having a nucleic acid sequence about 15 to 100 nucleotides in length and said first noncomplementary region optionally being at most about 5 kb in length; and
(b) a labeling reagent having a nucleic acid sequence complementary to said label reagent recognition sequence, wherein said label provides, directly or indirectly, a detectable signal; and
members of said second capturing set of reagents comprising:
(c) a plurality of capturing nucleic acid probes, different probes having a different second analyte complementary sequence and a second noncomplementary region comprising a capturing reagent recognition sequence, said second analyte complementary sequence having a nucleic acid sequence about 15 to about 100 nucleotides in length and said second noncomplementary region optionally being at most about 5kb in length; and
(d) a capturing reagent having a nucleic acid sequence complementary to said capturing reagent recognition sequence and capable of binding, directly or indirectly, with a separation means;
separating said label into a bound phase and an unbound phase by means of said separation means; and
detecting the amount of bound or unbound label as determinative of the presence of said analyte.

2. A method according to claim 1, wherein said nucleic acid sequence complementary to said capturing reagent recognition sequence is bound directly to said separation means, and said analyte, (a), (b), and (c) are combined together for a time sufficient for nucleic acid complexes to form, followed by the addition of said nucleic acid sequence complementary to said capturing reagent recognition sequence bound to said separation means.

3. A method according to claim 1, wherein said separation means is a solid support and wherein said capturing reagent is bound thereto.

4. An assay method for detecting a nucleic acid sequence in a sample, employing two sets of reagents: a first labeling set; and a second capturing set, said method comprising:
combining in a liquid medium under binding conditions for complementary nucleic acid sequences, said sample containing analyte in single-stranded form, members of said labeling set of reagents comprising:
(a) a plurality of labeling nucleic acid probes including a fluorescer or enzyme label, different probes having a different first analyte complementary sequence and a first noncomplementary region comprising a label reagent recognition sequence, said first analyte complementary sequence having a nucleic acid sequence about 15 to 100 nucleotides in length and said first noncomplementary region optionally being at most about 5kb in length; and
(b) a labeling reagent having a nucleic acid sequence complementary to said label reagent recognition sequence; and
members of said second capturing set of reagents comprising:
(c) a plurality of capturing nucleic acid probes, different probes having a different second analyte complementary sequence and a second noncomplementary region comprising a capturing reagent recognition sequence, said second analyte complementary sequence having a nucleic acid sequence about 15 to about 100 nucleotides in length and said noncomplementary region optionally being at most about 5kb in length; and
(d) a capturing reagent having a nucleic acid sequence complementary to said capturing reagent recognition sequence;
binding said capturing reagent to a solid support through a specific binding pair comprising a hapten and an antibody, either during or after said combining;
separating said label into a bound phase and an unbound phase by means of said solid support; and
detecting the amount of bound or unbound label as determinative of the presence of said analyte.

5. A kit for detecting a nucleic acid analyte comprising:
(1) members of a labeling set of reagents comprising:
(a) a plurality of labeling nucleic acid probes, different probes having a different first analyte complementary sequence and a first noncomplementary region comprising a label reagent recognition sequence, said first analyte complementary sequence having a nucleic acid sequence about 15 to 100 nucleotides in length and said first noncomplementary region optionally being at most about 5 kb in length; and
(b) a labeling reagent having a nucleic acid sequence complementary to said label reagent recognition sequence, wherein said label provides, directly or indirectly, a detectable signal; and
(2) members of a second capturing set of reagents comprising:
(c) a plurality of capturing nucleic acid probes, different probes having a different second analyte complementary sequence and a second noncomplementary region comprising a second capturing reagent recognition sequence having a nucleic acid sequence about 15 to about 100 nucleotides in length and said second noncomplementary region optionally being at most about 5 kb in length;
(d) a capturing reagent having a nucleic acid sequence complementary to said capturing reagent recognition sequence a first member of a specific binding pair conjugate; and
(e) support means conjugated to a second complementary member of said specific binding pair.

6. The use of the following in the manufacture of a kit for detecting a nucleic acid sequence:
(1) members of a labeling set of reagents comprising:
(a) a plurality of labeling nucleic acid probes, different probes having a different first analyte complementary sequence and a first noncomplementary region comprising a label reagent recognition sequence, said first analyte complementary sequence having a nucleic acid sequence about 15 to 100 nucleotides in length and said first noncomplementary region optionally being at most about 5 kb in length; and
(b) a labeling reagent having a nucleic acid sequence complementary to said label reagent recognition sequence, wherein said label provides, directly or indirectly, a detectable signal; and
(2) members of a second capturing set of reagents comprising:
(c) a plurality of capturing nucleic acid probes, different probes having a different second analyte complementary sequence and a second noncomplementary region comprising a second capturing reagent recognition sequence having a nucleic acid sequence about 15 to about 100 nucleotides in length and said second noncomplementary region optionally being at most about 5kb in length;
(d) a capturing reagent having a nucleic acid sequence complementary to said second recognition sequence and containing a first member of a specific binding pair conjugate; and
(e) support means conjugated to a second complementary member of said specific binding pair.

7. The use of a polynucleotide probe in an assay method according to any one of claims 1 to 4, wherein the probe has at least two nucleotides, at least one of which is given by the structure 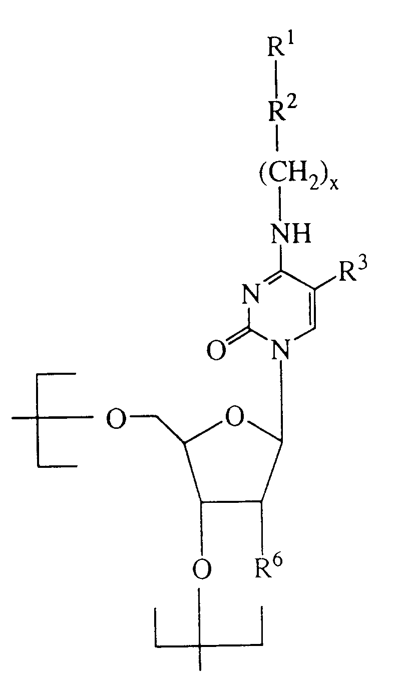 wherein R¹ is a reactive group derivatized with a detectable label, R² is an optional linking moiety including an amide, thioether or disulfide linkage or a combination thereof, R³ is selected from the group consisting of hydrogen, methyl, bromine, fluorine and iodine, R⁶ is H, OH or OR where R is a protecting group and x is an integer in the range of 1 to 8 inclusive.

8. The use according to claim 6 wherein a polynucleotide probe as defined in claim 7 is employed.

## Patentansprüche

1. Testverfahren zum Nachweis einer Nucleinsäuresequenz in einer Probe, wobei zwei Reagenziensätze eingesetzt werden: ein erster Markierungssatz und ein zweiter Einfangsatz, wobei das Verfahren folgende Schritte umfaßt:
Kombination in einem Flüssigmedium unter Bindungsbedingungen für komplementäre Paare der Probe, die den Analyt in Einzelstrangform enthält, Bestandteilen des Reagenzienmarkierungssatzes, umfassend:
(a) eine Vielzahl von Markierungsnucleinsäuresonden, unterschiedliche Sonden mit einer unterschiedlichen ersten Analytkomplementärsequenz und einem ersten Nichtkomplementärbereich, umfassend eine Markierungsreagenz-Erkennungssequenz, wobei die erste Analytkomplementärsequenz eine Nucleinsäuresequenz von etwa 15 bis 100 Nucleotiden Länge hat und der erste Nichtkomplementärbereich gegebenenfalls höchstens eine Länge von etwa 5 kb aufweist, und
b) ein Markierungsreagenz mit einer Nucleinsäuresequenz, die komplementär zu der Markierungsreagenz-Erkennungssequenz ist, wobei die Markierung direkt oder indirekt ein nachweisbares Signal liefert; und
Bestandteilen des zweiten Reagenzieneinfangsatzes, umfassend:
(c) eine Vielzahl von Einfangnucleinsäuresonden, unterschiedlichen Sonden mit einer unterschiedlichen zweiten Analytkomplementärsequenz und einem zweiten Nichtkomplementärbereich, umfassend eine Einfangreagenz-Erkennungssequenz, wobei die zweite Analytkomplementärsequenz eine Nucleinsäuresequenz von etwa 15 bis etwa 100 Nocleotiden Länge hat und der zweite Nichtkomplementärbereich gegebenenfalls höchstens eine Länge von etwa 5 kb aufweist, und
(d) ein Einfangreagenz mit einer Nucleinsäuresequenz, die komplementär zu der Einfangreagenz-Erkennungssequenz ist, und das zur direkten oder indirekten Bindung mit einem Trennmittel fähig ist;
Auftrennen des Markers in eine gebundene und eine nichtgebundene Phase mit Hilfe des Trennmittels; und Nachweis der Menge an gebundenem oder nicht-gebundenem Marker zur Bestimmung der Gegenwart des Analyten.

2. Verfahren nach Anspruch 1, wobei die Nucleinsäuresequenz, die komplementär zu der Einfangreagenz-Erkennungssequenz ist, direkt an das Trennmittel gebunden ist, und der Analyt, (a), (b) und (c) für einen Zeitraum zusammengegeben werden, der zur Bildung der Nucleinsäurekomplexe ausreicht, gefolgt von der Zugabe der Nucleinsäuresequenz, die komplementär zu der Einfangreagenz-Erkennungssequenz ist, die an das Trennmittel gebunden ist.

3. Verfahren nach Anspruch 1, wobei das Trennmittel ein fester Träger ist und wobei das Einfangreagenz daran gebunden ist.

4. Testverfahren zum Nachweis einer Nucleinsäuresequenz in einer Probe, wobei zwei Reagenziensätze eingesetzt werden: ein erster Markierungssatz und ein zweiter Einfangsatz, wobei das Verfahren folgende Schritte umfaßt:
Kombination in einem Flüssigmedium unter Bindungsbedingungen für komplementäre Nucleinsäuresequenzen der Probe, die den Analyt in Einzelstrangform enthält, Bestandteilen des Reagenzienmarkierungssatzes, umfassend:
(a) eine Vielzahl von Markierungsnucleinsäuresonden, einschließlich einer Fluoreszenz- oder Enzymmarkierung, unterschiedliche Sonden mit einer unterschiedlichen ersten Analytkomplementärsequenz und einem Nichtkomplementärbereich, umfassend eine Markierungsreagenz-Erkennungssequenz, wobei die erste Analytkomplementärsequenz eine Nucleinsäuresequenz von etwa 15 bis 100 Nucleotiden Länge hat und der erste Nichtkomplementärbereich gegebenenfalls höchstens eine Länge von etwa 5 kb aufweist, und
(b) ein Markierungsreagenz mit einer Nucleinsäuresequenz, die komplementär zu der Markierungsreagenz-Erkennungssequenz ist; und
Bestandteilen des zweiten Reagenzieneinfangsatzes, umfassend:
(c) eine Vielzahl von Einfangnucleinsäuresonden, unterschiedliche Sonden mit einer unterschiedlichen zweiten Analytkomplementärsequenz und einem zweiten Nichtkomplementärbereich, umfassend eine Einfangreagenz-Erkennungssequenz, wobei die zweite Analytkomplementärsequenz eine Nucleinsäuresequenz von etwa 15 bis etwa 100 Nucleotiden Länge hat und der Nichtkomplementärbereich gegebenenfalls höchstens eine Länge von etwa 5 kb aufweist, und
(d) ein Einfangreagenz mit einer Nucleinsäuresequenz, die komplementär zu der Einfangreagenz-Erkennungssequenz ist;
Bindung des Einfangreagenzes an einen festen Träger über ein spezifisches Bindungspaar, umfassend ein Hapten und einen Antikörper, entweder während oder nach der Kombination;
Auftrennen des Markers in eine gebundene und eine nichtgebundene Phase mit Hilfe des festen Trägers; und
Nachweis der Menge an gebundenem oder nichtgebundenem Marker zur Bestimmung der Gegenwart des Analyten.

5. Kit zum Nachweis eines Nucleinsäureanalyten, umfassend:
(1) Bestandteile eines Reagenzienmarkierungssatzes, umfassend:
(a) eine Vielzahl von Markierungsnucleinsäuresonden, unterschiedliche Sonden mit einer unterschiedlichen ersten Analytkomplementärsequenz und einem ersten Nichtkomplementärbereich, umfassend eine Markierungsreagenz-Erkennungssequenz, wobei die erste Analytkomplementärsequenz eine Nucleinsäuresequenz von etwa 15 bis 100 Nucleotiden Länge hat und der erste Nichtkomplementärbereich gegebenenfalls höchstens eine Länge von etwa 5 kb aufweist, und
(b) ein Markierungsreagenz mit einer Nucleinsäuresequenz, die komplementär zu der Markierungsreagenz-Erkennungssequenz ist, wobei die Markierung direkt oder indirekt ein nachweisbares Signal liefert; und
(2) Bestandteilen eines zweiten Reagenzieneinfangsatzes, umfassend:
(c) eine Vielzahl von Einfangnucleinsäuresonden, unterschiedliche Sonden mit einer unterschiedlichen zweiten Analytkomplementärsequenz und einem zweiten Nichtkomplementärbereich, umfassend eine zweite Einfangreagenz-Erkennungssequenz mit einer Nucleinsäuresequenz von etwa 15 bis etwa 100 Nucleotiden Länge, und wobei der zweite Nichtkomplementärbereich gegebenenfalls höchstens eine Länge von etwa 5 kb aufweist,
(d) ein Einfangreagenz mit einer Nucleinsäuresequenz, die komplementär zu der Einfangreagenz-Erkennungssequenz ist und einen ersten Bestandteil eines spezifischen Bindungspaarkonjugats enthält, und
(e) ein Trägermittel, das an einen zweiten komplementären Bestandteil des spezifischen Bindungspaares konjugiert ist.

6. Verwendung der folgenden Bestandteile für die Herstellung eines Kits zum Nachweis einer Nucleinsäuresequenz:
(1) Bestandteile eines Reagenzienmarkierungssatzes, umfassend:
(a) eine Vielzahl von Markierungsnucleinsäuresonden, unterschiedliche Sonden mit einer unterschiedlichen ersten Analytkomplementärsequenz und einem ersten Nichtkomplementärbereich, umfassend eine Markierungsreagenz-Erkennungssequenz, wobei die erste Analytkomplementärsequenz eine Nucleinsäuresequenz von etwa 15 bis 100 Nucleotiden Länge hat und der erste Nichtkomplementärbereich gegebenenfalls höchstens eine Länge von etwa 5 kb aufweist, und
(b) ein Markierungsreagenz mit einer Nucleinsäuresequenz, die komplementär zu der Markierungsreagenz-Erkennungssequenz ist, wobei die Markierung direkt oder indirekt ein nachweisbares Signal liefert; und
(2) Bestandteile eines zweiten Reagenzieneinfangsatzes, umfassend:
(c) eine Vielzahl von Einfangnucleinsäuresonden, unterschiedliche Sonden mit einer unterschiedlichen zweiten Analytkomplementärsequenz und einem zweiten Nichtkomplementärbereich, umfassend eine zweite Einfangreagenz-Erkennungssequenz mit einer Nucleinsäuresequenz von etwa 15 bis etwa 100 Nucleotiden Länge, und wobei der zweite Nichtkomplementärbereich gegebenenfalls höchstens eine Länge von etwa 5 kb aufweist,
(d) ein Einfangreagenz mit einer Nucleinsäuresequenz, die komplementär zu der zweiten Erkennungssequenz ist und einen ersten Bestandteil eines spezifischen Bindungspaarkonjugats enthält, und
(e) ein Trägermittel, das an einen zweiten komplementären Bestandteil des spezifischen Bindungspaares konjugiert ist.

7. Verwendung einer Polynucleotidsonde in einem Testverfahren nach einem der Ansprüche 1 bis 4, wobei die Sonde mindestens zwei Nucleotide aufweist, wobei mindestens eines die folgende Struktur aufweist 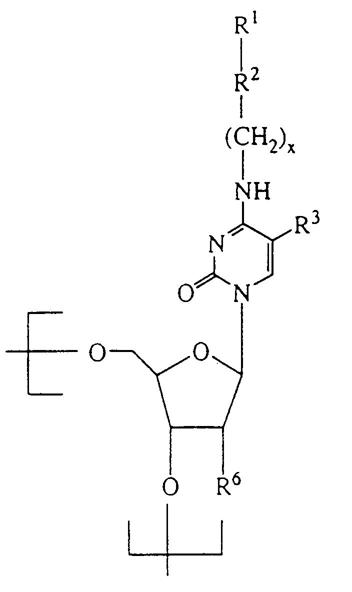 in der R¹ eine reaktive Gruppe ist, die mit einem nachweisbaren Marker derivatisiert ist, R² eine fakultative Verbindungseinheit ist, umfassend eine Amid-, Thioether- oder Disulfidverknüpfung oder eine Kombination davon, R³ ausgewählt ist aus Wasserstoff, einer Methylgruppe, einem Brom-, Fluor- und Iodatom, R⁶ ein Wasserstoffatom, eine OH-Gruppe oder ein OR-Rest ist, wobei R eine Schutzgruppe ist, und x eine ganze Zahl im Bereich von einschließlich 1 bis 8 ist.

8. Verwendung nach Anspruch 6, wobei eine Polynucleotidsonde gemäß der Definition in Anspruch 7 eingesetzt wird.

## Revendications

1. Procédé d'essai pour détecter une séquence d'acide nucléique dans un échantillon utilisant deux ensembles de réactifs :
un premier ensemble de marquage ; et un second ensemble de capture, ledit procédé comprenant :
la combinaison dans un milieu liquide, dans des conditions de liaison de paires complémentaires, dudit échantillon contenant la substance à analyser sous une forme monocaténaire, les éléments dudit ensemble de réactifs de marquage comprenant :
(a) plusieurs sondes d'acides nucléiques de marquage, les sondes différentes ayant une première séquence complémentaire de la substance à analyser et une première région non complémentaire comprenant une séquence de reconnaissance de réactif de marquage différentes ; ladite première séquence complémentaire de la substance à analyser ayant une séquence d'acide nucléique d'environ 15 à 100 nucléotides de longueur et ladite première région non complémentaire ayant facultativement une longueur d'au plus environ 5 kb ; et
(b) un réactif de marquage ayant une séquence d'acide nucléique complémentaire de ladite séquence de reconnaissance de réactif de marquage, où ledit marqueur fournit directement ou indirectement un signal détectable ; et
les éléments du second ensemble de réactifs de capture comprenant :
(c) plusieurs sondes d'acides nucléiques de capture, les sondes différentes ayant une seconde séquence complémentaire de la substance à analyser et une seconde région non complémentaire comprenant une séquence de reconnaissance de réactif de capture différentes, ladite seconde séquence complémentaire de la substance à analyser ayant une séquence d'acide nucléique d'environ 15 à environ 100 nucléotides de longueur et ladite seconde région non complémentaire étant facultativement longue d'au plus environ 5 kb ; et
(d) un réactif de capture ayant une séquence d'acide nucléique complémentaire de ladite séquence de reconnaissance du réactif de capture et capable de se lier, directement ou indirectement, avec un élément de séparation ;
la séparation dudit marqueur en une phase liée et une phase non liée au moyen dudit élément de séparation ; et
la détection de la quantité du marqueur lié ou non lié comme détermination de la présence de ladite substance à analyser.

2. Procédé selon la revendication 1, dans lequel ladite séquence d'acide nucléique complémentaire de ladite séquence de reconnaissance de réactif de capture est liée directement audit élément de séparation et la substance à analyser, (a), (b) et (c) sont combinés ensemble pendant un temps suffisant pour qu'il se forme des complexes d'acide nucléique, après quoi on ajoute ladite séquence d'acide nucléique complémentaire de ladite séquence de reconnaissance de réactif de capture liée audit élément de séparation.

3. Procédé selon la revendication 1, dans lequel ledit élément de séparation est un support solide et ledit réactif de capture lui est lié.

4. Procédé d'essai pour détecter une séquence d'acide nucléique dans un échantillon utilisant deux ensembles de réactifs :
un premier ensemble de marquage ; et un second ensemble de capture, ledit procédé comprenant :
la combinaison, dans un milieu liquide dans des conditions de liaison de séquences complémentaires d'acides nucléiques, dudit échantillon contenant la substance à analyser sous une forme monocaténaire, les éléments dudit ensemble de réactifs de marquage comprenant :
(a) plusieurs sondes d'acides nucléiques de marquage comprenant un marqueur fluorescent ou enzymatique, les sondes différentes ayant une première séquence complémentaire de la substance à analyser et une première région non complémentaire comprenant une séquence de reconnaissance de réactif de marquage différentes ; ladite première séquence complémentaire de la substance à analyser ayant une séquence d'acide nucléique d'environ 15 à 100 nucléotides de longueur et ladite première région non complémentaire ayant facultativement une longueur d'au plus environ 5 kb ; et
(b) un réactif de marquage ayant une séquence d'acide nucléique complémentaire de la dite séquence de reconnaissance de réactif de marquage ;
et
les éléments du second ensemble de réactifs de capture comprenant :
(c) plusieurs sondes d'acides nucléiques de capture, les sondes différentes ayant une seconde séquence complémentaire de la substance à analyser et une seconde région non complémentaire comprenant une séquence de reconnaissance de réactif de capture différentes, ladite seconde séquence complémentaire de la substance à analyser ayant une séquence d'acide nucléique d'environ 15 à environ 100 nucléotides de longueur et ladite région non complémentaire étant facultativement longue d'au plus environ 5 kb ; et
(d) un réactif de capture ayant une séquence d'acide nucléique complémentaire de ladite séquence de reconnaissance du réactif de capture ;
la liaison dudit réactif de capture à un support solide via une paire à liaison spécifique comprenant un haptène et un anticorps soit pendant, soit après ladite combinaison ;
la séparation dudit marqueur en une phase liée et une phase non liée au moyen dudit support solide ; et
la détection de la quantité du marqueur lié ou non lié comme détermination de la présence de ladite substance à analyser.

5. Nécessaire pour la détection d'une substance à analyser constituée d'acides nucléiques comprenant :
(1) les éléments d'un ensemble de réactifs de marquage comprenant :
(a) plusieurs sondes d'acides nucléiques de marquage, les sondes différentes ayant une première séquence complémentaire de la substance à analyser et une première région non complémentaire comprenant une séquence de reconnaissance de réactif de marquage différentes ; ladite première séquence complémentaire de la substance à analyser ayant une séquence d'acides nucléiques d'environ 15 à 100 nucléotides de longueur et ladite première région non complémentaire ayant facultativement une longueur d'au plus environ 5 kb ; et
(b) un réactif de marquage ayant une séquence d'acide nucléique complémentaire de la dite séquence de réactif de marquage, où ledit marqueur fournit directement ou indirectement un signal détectable ; et
(2) les éléments d'un second ensemble de réactifs de capture comprenant :
(c) plusieurs sondes d'acides nucléiques de capture, les sondes différentes ayant une seconde séquence complémentaire de la substance à analyser et une seconde région non complémentaire, comprenant une seconde séquence de reconnaissance de réactif de capture, différentes, ladite seconde séquence complémentaire de la substance à analyser ayant une séquence d'acide nucléique d'environ 15 à environ 100 nucléotides de longueur et ladite seconde région non complémentaire étant facultativement longue d'au plus environ 5 kb ; et
(d) un réactif de capture ayant une séquence d'acide nucléique complémentaire de ladite séquence de reconnaissance du réactif de capture et un premier élément d'un conjugué de paire à liaison spécifique ; et
(e) un élément support conjugué à un second élément complémentaire de ladite paire à liaison spécifique.

6. Utilisation des composants suivants dans la fabrication d'un nécessaire pour détecter une séquence d'acide nucléique :
(1) les éléments d'un ensemble de réactifs de marquage comprenant :
(a) plusieurs sondes d'acides nucléiques de marquage, les sondes différentes ayant une première séquence complémentaire de la substance à analyser et une première région non complémentaire comprenant une séquence de reconnaissance de réactif de marquage différentes ; ladite première séquence complémentaire de la substance à analyser ayant une séquence d'acide nucléique d'environ 15 à 100 nucléotides de longueur et ladite première région non complémentaire ayant facultativement une longueur d'au plus environ 5 kb ; et
(b) un réactif de marquage ayant une séquence d'acide nucléique complémentaire de la dite séquence de reconnaissance de réactif de marquage, où ledit marqueur fournit directement ou indirectement un signal détectable ; et
(2) les éléments d'un second ensemble de réactifs de capture comprenant :
(c) plusieurs sondes d'acides nucléiques de capture, les sondes différentes ayant une seconde séquence complémentaire de la substance à analyser et une seconde région non complémentaire comprenant une seconde séquence de reconnaissance de réactif de capture, différentes, ladite seconde séquence complémentaire de la substance à analyser ayant une séquence d'acide nucléique d'environ 15 à environ 100 nucléotides de longueur et ladite seconde région non complémentaire étant facultativement longue d'au plus environ 5 kb ; et
(d) un réactif de capture ayant une séquence d'acide nucléique complémentaire de ladite seconde séquence de reconnaissance et contenant un premier élément d'un conjugué d'une paire à liaison spécifique ; et
(e) un élément support conjugué à un second élément complémentaire de ladite paire à liaison spécifique.

7. Utilisation d'une sonde polynucléotidique dans un procédé d'essai selon l'une quelconque des revendications 1 à 4, où la sonde a au moins deux nucléotides dont au moins un répond à la structure 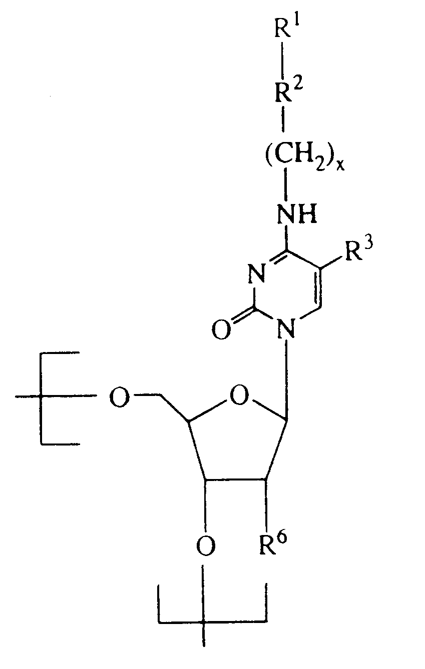 dans laquelle R¹ est un groupe réactif dérivatisé avec un marqueur détectable, R² est un fragment lieur facultatif comprenant une liaison amide, thioéther ou disulfure, ou une de leurs combinaisons, R³ est choisi dans le groupe constitué par un atome d'hydrogène, de brome, de fluor ou d'iode ou le groupe méthyle, R⁶ est H, OH ou OR, où R est un groupe protecteur et x est un entier dans la gamme de 1 à 8 inclusivement.

8. Utilisation selon la revendication 6, dans laquelle on utilise une sonde polynucléotidique telle que définie dans la revendication 7.
